# EUROPÄISCHE PATENTSCHRIFT

(11) **EP 4 233 807 B1**
(45) Veröffentlichungstag und Bekanntmachung des Hinweises auf die Patenterteilung: **07.05.2025**
(21) Anmeldenummer: 23156774.4
(22) Anmeldetag: 15.02.2023
(51) Int. Cl.: A61F 5/01, A01K 13/00

(54) **ANPASSBARE GAMASCHE, INSBESONDERE FÜR PFERDE**
ADJUSTABLE GAITER, IN PARTICULAR FOR HORSES
GUÊTRE ADAPTABLE, EN PARTICULIER POUR CHEVAUX

(30) Priorität: 18.02.2022 DE 102022201733
(43) Veröffentlichungstag der Anmeldung: 30.08.2023
(73) Patentinhaber: Engelke, Carsten, 29690 Lindwedel (DE)
(72) Erfinder: Engelke, Carsten, 29690 Lindwedel (DE)
(74) Vertreter: Taruttis, Stefan Georg

(56) Entgegenhaltungen:
- DE-A1- 4 029 120
- DE-B3- 102004 014 807
- DE-U1- 202010 008 448
- US-A1- 2019 021 893
- US-A1- 2020 022 861

## Beschreibung

Die vorliegende Erfindung betrifft eine Gamasche zur Verwendung als Stütze für ein Gelenk, bevorzugt für Tiere, insbesondere für Pferde, wobei die Gamasche um ein Fußgelenk oder Sprunggelenk anzuordnen ist, um das Gelenk zu stützen.

Die Gamasche zeichnet sich dadurch aus, dass sie bei der Anordnung am Gelenk eine präzise Passform annimmt und sich z.B. an die Oberfläche des Beins anpasst und anlegt, wobei die Gamasche überdies eine stabile und feste Stütze bildet und einen äußeren Schutz gegen mechanische Einwirkungen aufweist. Die Gamasche hat den Vorteil, dass sie Bewegungen eines umfassten Gelenks zulässt und bevorzugt sich solchen Bewegungen anpasst.

Die Gamasche ist auch zur Verwendung an Menschen geeignet, insbesondere im Bereich von Gelenken, z.B. am Fuß- oder Handgelenk.

Die DE 40 29 120 A1 beschreibt eine Gamasche, die zur Anpassung an einen Unterschenkel und Fuß mit Füllkörpern gefüllte und vakuumierbare Hüllen aufweist, die den Unterschenkel vollumfänglich umgreifen und die fest von Schalenteilen umspannt sind.

Die US 2019/0021893 A1 beschreibt eine orthopädische Stütze für ein Gelenk, bei der innenliegende Polster mittels vorstehender Pilzköpfe fest in Löcher starrer Rahmenelemente eingesetzt werden, mit einer elastischen Zwischenlage zwischen Polster und Rahmenelement, so dass der Schaft des Pilzkopfs durch die elastische Zwischenlage ragt und der Pilzkopf in die Ausnehmung des Rahmenelements ragt.

Die DE202010008448 U1 beschreibt eine Gamasche zum Schutz eines Beines eines Pferdes. Die DE102004014807 B3 beschreibt einen Sportstiefel und die US2020/022861 A1 beschreibt eine Gamasche zur Verwendung an Menschen.

Der Erfindung stellt sich die Aufgabe, eine Gamasche bereitzustellen, die auf einfache Weise um ein Fuß- oder Sprunggelenk eines Tiers, insbesondere eines Pferds angelegt werden kann, die sich einerseits über ihre im Wesentlichen vollständige Fläche passend anlegt und andererseits eine stabile Stütze bildet, ohne das Gelenk zu versteifen, und einen Schutz gegen äußere Einwirkung bildet. Bevorzugt soll die Gamasche eine Unterstützung für einen Bereich eines Gelenks bilden, die bei einer plötzlichen Belastung des Gelenks wirkt, aber in Abwesenheit plötzlicher Belastung formbar ist. Bevorzugt soll die Gamasche auf einfache Weise anzulegen sein und einfach zu reinigen sein.

Die Erfindung löst die Aufgabe mit den Merkmalen der Ansprüche und stellt insbesondere eine Gamasche bereit, die
als Einlage eine elastische Hülle, die eine formbare Masse enthält und optional ein Ventil aufweist,
wobei die Einlage zumindest einen vorstehenden Bereich als Haltevorsprung, bevorzugt an zumindest zwei gegenüberliegenden Bereichen ihrer Außenwand je zumindest einen vorstehenden Bereich als Haltevorsprung aufweist,
eine die Einlage umfassende gebogene Schale mit zwei gegenüberliegenden Schenkeln, die passend zu den Haltevorsprüngen der Einlage angeordnete Halteausnehmungen aufweist, die einen größeren Querschnitt als die Haltevorsprünge der Einlage aufweisen, um eine schwimmende Halterung von Haltevorsprüngen in je einer Halteausnehmung zu bilden, mit zumindest einer Lasche, die lösbar an gegenüberliegenden Schenkeln der Schale befestigbar ist, um den zwischen den gegenüberliegenden Kanten der Schenkel liegenden Freiraum zu überbrücken und die gegenüberliegenden Schenkel aufeinander zu zu spannen, wobei optional in der Schale, insbesondere endständig, ein relativ zur Schale bewegliches Schalensegment angeordnet ist, das zumindest eine Lasche aufweist, die lösbar an gegenüberliegenden Schenkeln des Schalensegments befestigbar ist.
weiter optional eine Zwischenlage, z.B. einschichtiger elastischer, optional aufgeschäumter Kunststoff, insbesondere einschichtiges aufgeschäumtes Neopren, in der Schale angeordnet ist,
aufweist oder daraus besteht.

Optional weist die Einlage genau zwei Haltevorsprünge auf, die weiter bevorzugt an einem ersten Endabschnitt der Einlage angeordnet sind, der sich über maximal 30%, bevorzugt maximal 25% oder maximal 20% oder maximal 10% bis 15% der Länge der Einlage entlang ihrer Längserstreckung und entlang der Längserstreckung der Schale erstreckt. Die Längserstreckung der Einlage liegt parallel zur Längserstreckung der Schale, bzw. parallel zur Längsachse der Schale, und senkrecht zum Querschnitt der Schale. Optional weist die Einlage an ihrem ersten Endabschnitt, der bevorzugt eingerichtet ist, am Röhrbein eines Pferds angeordnet zu werden, genau zwei Haltevorsprünge auf, die in passend am ersten Endabschnitt der Schale angeordnete Halteausnehmungen eingreifen, wobei der erste Endabschnitt der Schale eingerichtet ist, den ersten Endabschnitt der Einlage fest zu umfassen. Dabei liegt der erste Endabschnitt von Schale und Einlage bevorzugt an dem ersten Ende der Schale, das einen kleineren Innenquerschnitt aufweist und/oder gegenüber dem zweiten Ende der Schale und/oder der Einlage, an dem der zweite Endabschnitt der Schale und optional der zweite Endabschnitt der Einlage einen größeren Innenquerschnitt als am gegenüberliegenden jeweiligen ersten Endabschnitt aufweist. Die Schale weist bevorzugt an ihrem zweiten Endabschnitt einen größeren Innenquerschnitt als an ihrem ersten Endabschnitt auf, wobei der zweite Endabschnitt vorgesehen ist, mit Abstand von der Einlage um den Bereich eines Gelenks, insbesondere um das Fesselgelenk eines Pferds angeordnet zu werden, während das erste Ende der Schale vorgesehen ist, anliegend an den ersten Endabschnitt der Einlage angeordnet zu werden, der eingerichtet ist, am Röhrbein anzuliegen. Der zweite Endabschnitt der Schale ist eingerichtet, in einen Abstand von der Einlage, insbesondere in einen Abstand vom zweiten Endabschnitt der Einlage beweglich bzw. mit Abstand vom zweiten Endabschnitt der Einlage angeordnet zu sein, so dass sich der zweite Endabschnitt der Einlage vom zweiten Endabschnitt der Schale weg bewegen kann, z.B. bei einer Bewegung im Fesselgelenk. Daher ist der zweite Endabschnitt der Schale nicht fest mit dem zweiten Endabschnitt der Einlage verbunden, sondern der zweite Endabschnitt der Schale ist um die am ersten Endabschnitt von Schale bzw. Einlage in die Halteausnehmungen eingreifenden Haltevorsprünge gegen den zweiten Endabschnitt der Einlage schwenkbar angelenkt und bevorzugt schwimmend verschieblich geführt.

Die erfindungsgemäße Manschette ist durch den am zweiten Endabschnitt der Schale größeren Innenquerschnitt als am ersten Endabschnitt eingerichtet, dass die Einlage an ihrem ersten Endabschnitt durch den ersten Endabschnitt der Schale fest an einem Röhrbein anliegt und der zweite Endabschnitt der Einlage bei Bewegung im Fesselgelenk in einen Abstand vom zweiten Endabschnitt der Schale schwenkbar ist. Die Einlage kann mit ihrer formbaren Masse, insbesondere durch Verfestigen mittels Vakuumierens einer durch lose Partikel in der Hülle gebildeten formbaren Masse, passend an Röhrbein und/oder Fesselgelenk in einer Stellung des Fesselgelenks angepasst werden. Dabei überdeckt die Schale die Einlage und die Einlage ist zumindest im zweiten Endabschnitt schwenkbar zur Schale geführt.

Der Innenquerschnitt der Schale an deren zweiten Endabschnitt kann z.B. einen um 10 % bis 20% und/oder einen um 2 bis 10 cm oder bis 8 cm größeren Durchmesser als der erste Endabschnitt aufweisen.

Die Schenkel der Schale bilden vorzugsweise einen U-förmigen Querschnitt der Schale. Die Schale kann entlang einer Mittelebene symmetrische Schenkel aufweisen bzw. daraus bestehen. Die Hülle kann einen gebogenen rechteckigen Umfang aufweisen, bevorzugt einen Querschnitt aufweisen, der zwei gegenüberliegende Schenkel hat, die ausschließlich innerhalb der Schale bzw. auf der Innenseite der Schale angeordnet sind und nicht über die Schale vorstehen.

Die Halteausnehmungen können Durchbrechungen der Schale sein, durch die die vorstehenden Bereiche, die die Haltevorsprünge bilden, hindurch ragen. Optional sind die Halteausnehmungen Einbuchtungen, die sich von der konkaven Innenseite der Schale erstrecken, wobei die gegenüberliegende konvexe Außenseite der Schale eine geschlossene Oberfläche hat, die optional im Bereich der Ausnehmungen vorgewölbt sein kann.

Bevorzugt sind die vorstehenden Bereiche der Hülle, die die Einlage bildet, und die passend ausgerichteten Halteausnehmungen der Schale entlang der einander gegenüberliegenden Kanten der Schenkel angeordnet, z.B. zumindest je zwei, zumindest je drei, zumindest je vier entlang jeder der beiden gegenüberliegenden Kanten.

Die formbare Masse ist generell eine, die bei Umgebungstemperatur und Körpertemperatur, z.B. zwischen 5 und 40 °C, einen formbaren Zustand und einen festen Zustand annehmen kann, wobei der Zustand einstellbar ist oder die Masse den Zustand abhängig von der äußeren Belastung einnimmt.

Die formbare Masse kann von Partikeln gebildet sein, z.B. Kunststoffkugeln, z.B. aus Kunststoffschaum, insbesondere aufgeschäumtem Polystyrol, aufgeschäumter Keramik oder massiven oder hohlen Partikel, bevorzugt Kugeln. Die Partikel können in einem formbaren Zustand lose gegeneinander beweglich sein oder beweglich in einer Mischung mit einem dilatanten Fluid vorliegen. Die Partikel können z.B. einen Durchmesser von 1 bis 10 mm, bevorzugt 2 bis 7 oder bis 5 mm aufweisen.

Die Hülle, die die Einlage bildet, ist luftdicht und in der Ausführungsform, in der die formbare Masse durch Partikel gebildet ist, durch ein Ventil, das vorzugsweise durch eine zusätzliche Durchbrechung der Schale zugänglich ist, evakuierbar, so dass die in der Hülle enthaltenen Partikel nach Vakuumierung eine feste Packung als festen Zustand bilden, während die Partikel bei offenem Ventil gegeneinander beweglich sind und eine lose Schüttung bilden, die sich an die Hülle anpasst. Generell bevorzugt ist das Ventil nicht fest mit der Schale verbunden, sondern tritt z.B. durch eine Öffnung der Schale, die größer als das Ventil ist. Vorzugsweise ist das Ventil verschieblich, auch als schwimmend bezeichnet, in der Schale gehalten, z.B. verschieblich entlang zweier Achsen parallel zur Oberfläche der Schale, jeweils um zumindest 1 mm, bevorzugt zumindest 2 mm oder zumindest 3 mm, z.B. um bis zu 15 mm, bis zu 12 mm, bis zu 9 mm oder bis zu 6 mm verschieblich.

Die formbare Masse kann von einem dilatanten Fluid gebildet sein, z.B. borhaltiges Polydimethylsiloxan, auch als Borsilikon-Kitt bekannt, oder einer borsäurehaltigen wässrigen Lösung von Polyvinylalkohol. Ein dilatantes Fluid als formbare Masse in zumindest einem Segment der Hülle hat den Vorteil, dass es bei stetiger Belastung verformbar ist und in diesem formbaren Zustand an ein Gelenk anpassbar ist, und dass das dilatante Fluid bei plötzlicher Belastung steifer wird, einen festen Zustand annimmt und eine feste Unterstützung für ein anliegendes Gelenk bildet.

Die Einlage ist bevorzugt ausschließlich mit ihren vorstehenden Bereichen, die Haltevorsprünge bilden und in die passend ausgerichteten Halteausnehmungen der Schale vorstehen, wobei die Halteausnehmungen einen größeren Querschnitt als die Haltevorsprünge aufweisen, verschieblich, auch als schwimmend bezeichnet, in der Schale gehalten, z.B. verschieblich entlang zweier Achsen parallel zur Oberfläche der Schale, jeweils um zumindest 1 mm, bevorzugt zumindest 2 mm oder zumindest 3 mm, z.B. um bis zu 15 mm, bis zu 12 mm, bis zu 9 mm oder bis zu 6 mm verschieblich. Die Einlage ist daher einfach mit der Schale zu verbinden und auch einfach von der Schale zu lösen, z.B. für eine Reinigung beider. Durch die Haltevorsprünge, die in Halteausnehmungen größeren Querschnitts eingreifen, ist die Einlage verschieblich bzw. schwimmend an der Schale gelagert, insbesondere bevorzugt dadurch, dass ausschließlich am ersten Endabschnitt der Einlage angeordnete Haltevorsprünge in am ersten Endabschnitt der Schale angeordnete Halteausnehmungen verschieblich eingreifen.

Optional kann die Einlage, z.B. ein Segment der Hülle, mit einer lösbaren Haftverbindung, z.B. einem Klettverschluß, mit der Schale verbunden sein, z.B. ausschließlich entlang oder quer zu einer Mittelebene, so dass die beiden beidseitig der Haftverbindung liegenden Bereiche der Einlage frei in der Schale beweglich sind und nur mit ihren Haltevorsprüngen in die Halteausnehmungen der Schale vorstehen.

Erfindungsgemäss ist zumindest einer, bevorzugter zumindest zwei der Haltevorsprünge federnd schwimmend in je einer Halteausnehmung gehalten,. mittels Federelementen, die sich in der Halteausnehmung bis zu einem Haltevorsprung erstrecken. Dabei kann an der Schale zumindest ein Federelement angebracht sein, bevorzugt zwei oder drei Federelemente, von denen jedes eine Halteaufnahme an oder in der Halteausnehmung hält, wobei die Halteaufnahme zur Verrastung oder Verklemmung eines Haltevorsprungs eingerichtet ist. Die Federelemente können mit einer ringförmigen Halteaufnahme verbunden sein oder diese bilden, so dass ein Haltevorsprung in die Halteaufnahme geklemmt werden kann. Eine federnde und schwimmende Halterung jedes Haltevorsprungs in einer Halteausnehmung, z.B. in einer federnd in der Halteausnehmung gehaltenen Halteaufnahme, hat den Vorteil, dass sich die Einlage innerhalb der Schale bewegen kann, z.B. um den Abstand zwischen Halteausnehmung und Haltevorsprung, und die Einlage durch die Federwirkung in eine Ruhelage des Federelements belastet wird. Bevorzugt ist eine Halteaufnahme in Ruhelage des Federelements, in der die Einlage nicht entlang der Längserstreckung der Schale belastet wird, mittig in der Halteausnehmung angeordnet. Ein Federelement kann z.B. als Speiche, gerade, geknickt oder bogenförmig, z.B. radial oder in einem Winkel zur Radialen zwischen Halteausnehmung und Halteaufnahme angeordnet, aus Kunststoff oder Federstahl, ausgebildet sein, die sich zwischen Halteausnehmung und Halteaufnahme erstreckt.

Ein Schalensegment, das generell optional ist, ist bevorzugt am zweiten Endabschnitt der Schale angeordnet. Optional weisen die Einlage und die Schale sowie das optionale Schalensegment nur glatte Oberflächen auf, wobei optional kein zwischenliegender Stoff wie z.B. ein Fleece, ein Gel oder eine Schmierverbindung zwischen Hülle und Schale bzw. zwischen Hülle und Gelenk angeordnet ist. Denn es hat sich gezeigt, dass bei Befestigung der Gamasche am Gelenk kaum Reibungskräfte zwischen Hülle und Schale wirken, sodass kein die Reibungswirkung reduzierender Stoff erforderlich ist und die Gamasche den zusätzlichen Vorteil hat, dass sie einfach zu reinigen ist. Alternativ kann zwischen der Hülle, die die Einlage bildet, und der Schale ein zwischenliegender Stoff, z.B. Fleece oder Schaumstoff, angeordnet sein.

Die Einlage kann einteilig sein und sich über die Länge der Schale und des optionalen Schalensegments erstrecken, so dass ein Abschnitt der Einlage von der Schale eingefasst ist und ein Abschnitt, insbesondere ein endständiger Abschnitt der Einlage von dem optionalen Schalensegment eingefasst ist.

Bevorzugt weist die Einlage senkrecht zu ihrer Längserstreckung eine Breite auf, die sich um 180° bis 230° oder bis 210° oder bis 180°, z.B. von 180 bis 200 ° innerhalb des von der Einlage und/oder von der Schale aufgespannten Querschnitts erstreckt, so dass die Einlage nicht den vollständigen Umfang von 360° eines Beins, insbesondere eines Röhrbeins oder Arms umfasst, sondern nur einen Anteil von maximal 230°, insbesondere zur Verwendung zur Anordnung der Einlage gegen die Rückseite eines Beins, insbesondere eines Röhrbeins eines Pferds, um die entlang des Beins verlaufende Sehne zu stützen, z.B. die oberflächliche und tiefe Beugesehne eines Pferds. Die Schale kann sich um den gleichen oder einen größeren Anteil des Umfangs eines Beins erstrecken, bevorzugt um zumindest 10 bis 30° mehr als die Einlage, so dass die Schale die Einlage vollständig abdeckt.

Optional ist die Hülle, die die Einlage bildet, in miteinander verbundene Einlagensegmente unterteilt, die z.B. dadurch erzeugt sind, dass streifenförmige Bereiche der Innenwand und der Außenwand der Hülle, die sich gegenüberliegen, miteinander verschweißt sind, bevorzugt etwa parallel oder in einem Winkel von maximal 30° oder maximal 20° zur Längserstreckung der Schale. Dabei sind die Einlagensegmente z.B. dadurch miteinander verbunden, dass die Verschweißung nicht kontinuierlich ist, z.B. als Abfolge von beabstandeten punktförmigen oder strichförmigen Schweißstellen, und/oder die Verschweißung zumindest einen Bereich zwischen Einlagensegmenten offenlässt, z.B. angrenzend an die Umfangskante.

Optional sind Einlagensegmente, die durch Segmente der Hülle gebildet werden, voneinander beabstandet und miteinander durch eine Leitung, z.B. durch einen Bereich der Hülle, insbesondere Unterbrechungen in Schweißnähten gebildet, miteinander verbunden und weisen nur ein gemeinsames Ventil auf.

Die Einlagensegmente, die durch Segmente der Hülle gebildet werden, können voneinander beabstandet sein, getrennte Innenvolumina und jeweils ein separates Ventil aufweisen, oder gasdicht und ohne Ventil sein, wenn die formbare Masse ein dilatantes Fluid, optional in Mischung mit losen Partikeln aufweist oder daraus besteht. Voneinander beabstandete Segmente der Hülle, die jeweils Partikel enthalten und optional durch jeweils ein separates Ventil vakuumierbar sind oder kein Ventil aufweisen, können miteinander verbunden sein, z.B. an einem gemeinsamen Folienabschnitt angebracht sein, z.B. durch einen Abschnitt einer Kunststofffolie, in der die Segmente gebildet sind, oder die Segmente können vollständig voneinander getrennt ausgebildet sein. Segmente einer Hülle bilden jeweils Segmente der Einlage, so dass ein Segment von der Schale umfasst ist und ein weiteres Segment der Einlage von einem Schalensegment umfasst ist. Optional kann die Einlage zumindest ein Segment mit weniger formbarer Masse als angrenzende Segmente oder ohne formbare Masse aufweisen, z.B. ein zentrales oder asymmetrisch zu einer Mittellängsachse angeordnetes Einlagensegment mit mehr formbarer Masse, mit weniger formbarer Masse oder ohne formbare Masse, das optional an seinem Umfang, z.B. allseitig oder beidseitig an zumindest ein Einlagensegment mit unterschiedlichem Gehalt und/oder im Übrigen an Einlagensegmente mit jeweils gleichem Gehalt an formbarer Masse angrenzt, z.B. um in dem Bereich des Einlagensegments mit weniger formbarer Masse einen größeren Innenquerschnitt zu bilden, in den sich z.B. druckempfindliche Bereiche eines Arms oder Beins, insbesondere eines Fuß- oder Sprunggelenks erstrecken können. Dabei ist der Gehalt an formbarer Masse der Gehalt pro cm³ des maximalen Volumens des Einlagensegments oder der Gehalt pro cm² der Fläche des Einlagensegments, die sich parallel zur Schale erstreckt.

Generell können Einlagensegmente, aneinander angrenzend oder beabstandet, in einer Reihe entlang der Längsachse der Schale angeordnet sein. Optional enthält ein von einem endständigen Bereich der Schale oder von einem Schalensegment umfasstes Segment der Hülle, das von anderen Segmenten der Hülle getrennt ist, ein dilatantes Fluid, optional in Mischung mit Partikeln. Ein Segment der Hülle, das ein dilatantes Fluid, optional in Mischung mit Partikeln, enthält, kann luftdicht sein und ohne Ventil. Ein mit dilatantem Fluid, optional in Mischung mit Partikeln, gefülltes Segment der Hülle kann generell eine Einlage bilden, die zumindest einen eigenen Haltevorsprung aufweist, der in eine passend angeordnete Halteausnehmung der Schale oder des Schalensegments ragt, wobei die Halteausnehmung einen gleich großen Querschnitt wie der Haltevorsprung aufweist oder einen größeren Querschnitt als der Haltevorsprung. Alternativ kann ein Segment der Hülle, das ein dilatantes Fluid enthält, optional in Mischung mit Partikeln, insbesondere wenn dieses Segment der Hülle nicht mit anderen Segmenten der Hülle verbunden ist, an einem endständigen Bereich der Schale oder an einem Schalensegment festgelegt sein, z.B. mittels einer Haftverbindung, optional mit zwischenliegender elastischer Polsterschicht, z.B. Schaumstoff, der z.B. aufgeschäumtes Neopren sein kann.

Optional wird die Einlage durch eine vakuumierbare Hülle, z.B. aus Kunststofffolie gebildet, innerhalb derer eine mit Partikeln gefüllte gasdurchlässige Innenlage, z.B. aus Stoff, gewebt oder Flies, angeordnet ist. Die Innenlage kann in Segmente unterteilt sein, die z.B. durch miteinander verbundene Bereiche der Innenlage wie z.B. Abnäher oder Schweißnähte unterteilt sind, die mit Partikeln gefüllt sind, jeweils zu einem gleichen oder unterschiedlichen Volumenanteil der Segmente. Bevorzugt verlaufen Segmente der Innenlage bzw. die sie trennenden, miteinander verbundenen Bereiche der Innenlage parallel zur Längserstreckung der Einlage.

Vorzugsweise wird die Einlage an ihrer umlaufenden Umfangskante von der sie umfassenden Schale um zumindest 1 mm, bevorzugt zumindest 2 mm oder zumindest 3 mm, z.B. bis zu 15 mm, bis zu 12 mm, bis zu 9 mm oder bis zu 6 mm überragt. Dann hat die Gamasche den Vorteil, dass die Hülle auch nach der Evakuierung einer Bewegung des Gelenks ungestört folgen kann und insbesondere nicht am Gelenk reibt.

Das optionale Schalensegment weist bevorzugt eine Außenwand auf, die angrenzend an, optional mit Abstand in der Schale angeordnet ist, so dass das Schalensegment von der Schale umfasst und in der Schale beweglich ist, insbesondere gegen eine Längsachse bzw. um eine Querachse der Schale drehbar angeordnet ist. Das Schalensegment kann Haltevorsprünge aufweisen, die sich z.B. etwa senkrecht zur Längsachse der Schale über die Außenwand des Schalensegments vorstehen, wobei diese Haltevorsprünge in passend angeordnete Ausnehmungen der Schale eingreifen. Bevorzugt haben die Ausnehmungen der Schale einen größeren Querschnitt als die Haltevorsprünge des Schalensegments, um das Schalensegment längs der Längsachse der Schale verschieblich und/oder gegen die Längsachse bzw. um eine Querachse der Schale drehbar zu führen.

Das optionale Schalensegment kann dadurch mit der Schale verbunden sein, dass es mit der Einlage und/oder mit einer Zwischenlage verbunden ist, die innerhalb der Schale angeordnet ist.

Das Schalensegment und die daran fixierbare Lasche umfassen die Einlage, bevorzugt an einem ihrer endständigen Abschnitte, umfänglich, so dass das Schalensegment eine gegenüber der Schale bewegliche und in sich starre Umfassung eines bevorzugt endständigen Abschnitts der Einlage bildet.

Die optionale Zwischenlage ist bevorzugt zwischen einerseits der Einlage und andererseits der Schale und einem darin angeordneten Schalensegment angeordnet, alternativ kann die optionale Zwischenlage zwischen einerseits der Einlage und dem Schalensegment und andererseits der Schale angeordnet sein. Weiter alternativ kann die Zwischenlage an der Oberfläche der Einlage angeordnet sein, die der Schale gegenüberliegt.

Bevorzugt weist die Zwischenlage passend zu den Haltevorsprüngen der Einlage angeordnete Ausnehmungen auf, in die die Haltevorsprünge eingreifen. Die Haltevorsprünge, die in die Halteausnehmungen der Schale eingreifen, halten die Einlage und die optionale Zwischenlage an der Schale. Alternativ kann die Zwischenlage fest mit der Einlage oder mit der Schale und/oder dem Schalensegment verbunden sein, z.B. an der der Schale zugewandten Außenseite der Einlage, z.B. mit einer festen Verbindung, z.B. Klebverbindung oder Schweißverbindung, oder einer lösbaren Verbindung, z.B. einem Klettverschluß, befestigt sein.

Die Gamasche ist zur Verwendung als Stütze eines Fuß- oder Sprunggelenks z.B. eines Menschen, vorzugsweise eines Tiers, insbesondere eines Pferdes, geeignet, wobei die Gamasche mit geöffnetem Ventil an das Fuß- oder Sprunggelenk angelegt wird, z.B. mit leichtem Druck, bis die Hülle, die die Einlage bildet, anliegt, anschließend die Hülle durch das Ventil vakuumiert wird, wodurch sich die lose Schüttung der Partikel zu einer festen Packung verdichtet und sich die vorstehenden Bereiche in den Ausnehmungen der Schale bewegen, und anschließend die gegenüberliegenden Schenkel der Schale mittels der zumindest einen Lasche aufeinander zu belastet werden, um die Schale an die Einlage zu legen, wobei die Schale und das optionale Schalensegment relativ zur Einlage beweglich sind oder fest an der Einlage anliegen, wobei das Schalensegment beweglich an der Schale geführt ist. Bevorzugt weist die Gamasche zumindest zwei, bevorzugter zumindest drei Laschen auf, z.B. bis zu sechs oder bis zu fünf oder vier Laschen, die sich über den Abstand der gegenüberliegenden Schenkel der Schale erstrecken und diese aufeinander zu belasten können.

Optional bildet jeweils eine Schale mit zumindest zwei Hüllen ein System, bei dem die Hüllen gegeneinander austauschbar sind und jede Hülle eine unterschiedliche Füllung von formbarer Masse, z.B. aus losen Partikeln und/oder dilatantem Fluid, aufweist. Die Füllung kann z.B. nach Art der Partikel oder Gehalt der Hülle an Partikeln und Art und Volumen des dilatanten Fluids unterschiedlich sein. In dieser Ausführungsform hat die Gamasche den Vorteil, dass eine Schale durch Auswahl und Anordnung einer der Hüllen in der Schale an ein Fuß- oder Sprunggelenk anpassbar ist.

Die Erfindung wird nun mit Bezug auf die Figuren beschrieben, die die Gamasche schematisch in
- Fig. 1 in Explosionszeichnung einer Hülle und einer Schale,
- Fig. 2 als in der Schale angeordnete Hülle,
- Fig. 3a bis 3c als einzelne und zusammengesetzte Teile in einer Ausführungsform,
- Fig. 4a bis 4c als einzelne und zusammengesetzte Teile in einer weiteren Ausführungsform,
- Fig. 5a bis 5e als einzelne und zusammengesetzte Teile in einer weiteren Ausführungsform,
- Fig. 6a bis 6e als einzelne und zusammengesetzte Teile in einer noch weiteren Ausführungsform,
- Fig. 7 eine Ausführungsform einer Halteausnehmung mit Federelementen, die mit einer Halteaufnahme verbunden sind, und
- Fig. 8 schematisch eine in Einlagensegmente unterteilte Einlage
zeigen.

In den Figuren bezeichnen gleiche Bezugsziffern funktionsgleiche Teile.

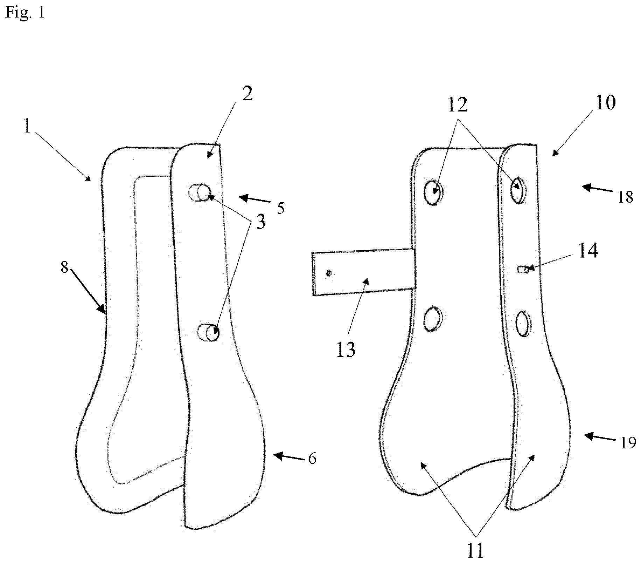

Fig. 1 zeigt eine Einlage 1, die an gegenüberliegenden Bereichen ihrer Außenwand 2 jeweils zwei vorstehende Bereiche 3 aufweist, wobei nur die vorstehenden Bereiche, die die Haltevorsprünge 3 bilden, des in Fig. 1 vorderen bzw. rechten Bereichs der Außenwand 2 gezeigt sind. Einer der Haltevorsprünge kann ein Ventil 4 sein. Weiter ist eine gebogene Schale 10 mit zwei gegenüberliegenden Schenkeln 11 gezeigt, die passend zu den Haltevorsprüngen 3 der Einlage 1 angeordnete Halteausnehmungen 12 aufweist, die einen größeren Querschnitt als die vorstehenden Haltevorsprünge 3 aufweisen. Die Einlage 1 wird von der Umfangskante 8 begrenzt. Stellvertretend für zumindest zwei Laschen ist eine Lasche 13 gezeigt, die an den gegenüberliegenden Schenkeln 11 der Schale 10 mittels eines hier gezeigten Vorsprungs 14 lösbar befestigbar ist. Der erste Endabschnitt 5 der Einlage 1 liegt am ersten Endabschnitt 18 der Schale 1 an, der dem ersten Endabschnitt 5 gegenüberliegende zweite Endabschnitt 6 der Einlage 1 ist im Bereich des zweiten Endabschnitts 19 der Schale 1 angeordnet. In Ausführungsformen, in denen die Einlage 1 genau einen Haltevorsprung 3 oder, bevorzugt, zwei im ersten Endabschnitt 5 der Einlage 1 im gleichen Bereich entlang der Längsachse der Schale 10, die generell bevorzugt gleich der Längsachse der Einlage 1 ist, angeordnete Haltevorsprünge 3 aufweist, die in am ersten Endabschnitt 18 der Schale 11 angeordneten Halteausnehmungen 12 verschieblich geführt sind, ist der dem ersten Endabschnitt 18 gegenüberliegende zweite Endabschnitt 19 der Schale 1 in einen Abstand vom zweiten Endabschnitt 6 der Einlage 1 weg schwenkbar.

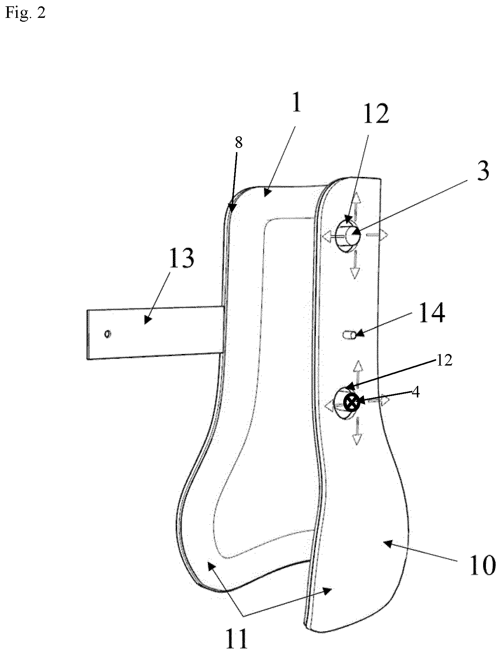

Fig. 2 zeigt die in der Schale 10 angeordnete Einlage 1, deren vorstehende Haltevorsprünge 3 durch die hier als Durchbrechungen gezeigten Halteausnehmungen 12 ragen, sodass die Einlage 1 entlang der hier gezeigten hohlen Pfeile verschieblich in der Schale 10 gehalten ist. Ein Ventil 4, durch das die als Hülle ausgebildete Einlage 1 evakuiert oder belüftet werden kann, kann durch eine Halteausnehmung 12 ragen Die Lasche 13 kann durch den Vorsprung 14 lösbar befestigt werden, um den zwischen den Schenkeln 11 der Schale 10 liegenden Freiraum zu überbrücken und die gegenüberliegenden Schenkel 11 aufeinander zu zu spannen.

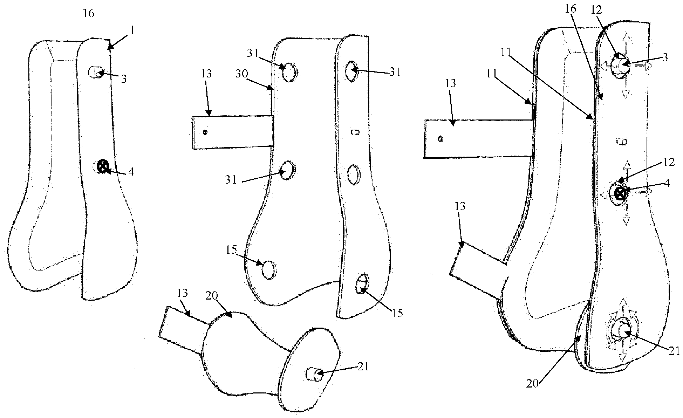

Fig. 3 zeigt eine bevorzugte Ausführungsform, bei der eine einstückige Einlage 1 (Fig. 1A), die von einer einteiligen Hülle gebildet wird, entlang ihrer vollständigen Länge von einer Schale 10 umfasst wird. Die Fig. 3A zeigt die Schale 10 mit in gegenüberliegenden Kantenbereichen paarweise angeordneten Halteausnehmungen 12, sowie in einem endständigen Abschnitt der Schale 10 angeordnete Ausnehmungen 15, die passend zu Haltevorsprüngen 21 eines Schalensegments 20 angeordnet sind. Das Schalensegment 20, in Fig. 3A getrennt von der Schale 10 gezeigt, kann mit seinen Haltevorsprüngen 21 in die Ausnehmungen 15 der Schale 10 eingreifen, um eine gegen die Längsachse 16 der Schale schwenkbar gelagert zu sein. Das Schalensegment 20 weist eine Lasche 22 auf, um den Abstand zwischen den gegenüberliegenden Schenkeln 23 des Schalensegments 20 zu überdecken, wenn sie an beiden Schenkeln 23 festgelegt ist.

Fig. 3C zeigt die Gamasche, die mit der Schale 10 eine Einlage 1 umfasst, mit einem zwischen der Einlage 1 und der Schale 10 an der Schale 10 beweglich gelagerten Schalensegment 20. Die Beweglichkeit der Haltevorsprünge 3 der Einlage 1 in Halteausnehmungen 12 der Schale 10, sowie die Beweglichkeit der Haltevorsprünge 21 des Schalensegments 20 in Ausnehmungen 15 der Schale 10 sind durch die hohlen Pfeile in Fig. 3C angedeutet. Die Schale 10 kann mittels der Lasche 13 um die Einlage 1 geschlossen werden, und das Schalensegment 20 kann mittels seiner Lasche 22 separat um die Einlage 1 geschlossen werden.

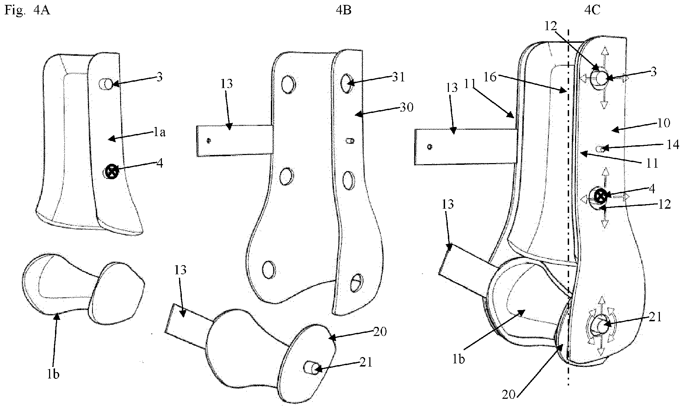

Fig. 4a bis 4c zeigen eine Ausführungsform, in der die Einlage 1 in zwei Einlagesegmente 1a, 1b unterteilt ist, von denen jedes mittels eines separaten Ventils 4 evakuierbar ist. Ein Einlagesegment 1b ist passend zur Innenseite eines Schalensegments 12 ausgebildet, das das Einlagesegment 1b umfasst. Das andere Einlagesegment 1a ist passend zu dem Bereich der Innenseite der Schale 10 ausgebildet, der nicht vom Schalensegment 20 überdeckt wird, das beweglich in der Schale 10 angeordnet wird. In dieser Ausführungsform ist die Einlage, die in zwei Einlagesegmente 1a, 1b unterteilt ist, auch bei evakuierten Schalensegmenten entsprechend der Bewegung des Schalensegments 20 in der Schale 10 beweglich und daher gut an die Bewegung eines von der Gamasche umfassten Gelenks angepasst.

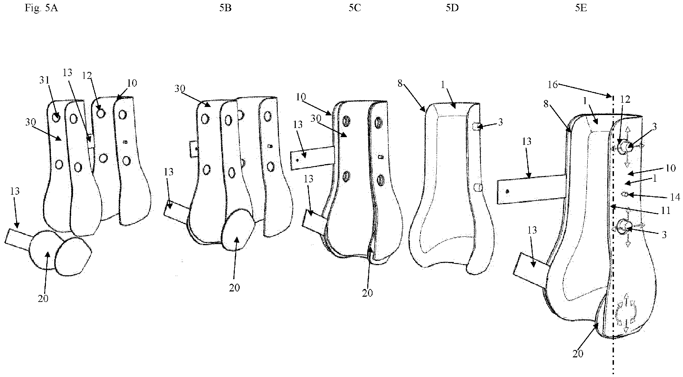

Fig. 5a bis 5e zeigen eine Ausführungsform, in der eine Zwischenlage 30 zwischen einerseits einer Einlage 1 und andererseits einer Schale 10 und einem Schalensegment 20 angeordnet ist. Die Zwischenlage 30 weist Ausnehmungen 31 auf, die passend zu Halteausnehmungen 12 der Schale 10 angeordnet sind, so dass Haltevorsprünge 3 der Einlage 1 (Fig. 5d) durch die Ausnehmungen 31 und die Halteausnehmungen 12 angeordnet werden, um die Zwischenlage 30 zwischen der Schale 10 und der Einlage 1 zu halten (Fig. 5e). Das Schalensegment 20 ist bevorzugt an der Zwischenlage 30 befestigt, z.B. mittels einer Haft- oder Klettverbindung. Alternativ kann das Schalensegment 30 lose zwischen der Zwischenlage 30 und der Schale 10 angeordnet sein und durch seine Lasche 22, wenn diese die Einlage 1 umfasst, mit Reibschluß gegen die Zwischenlage 30 belastet oder geklemmt sein.

Auch in dieser Ausführungsform ist das Schalensegment 30 zur Schale 10 beweglich in dieser angeordnet und umfasst mit der Lasche 22 einen endständigen Abschnitt der Einlage 1.

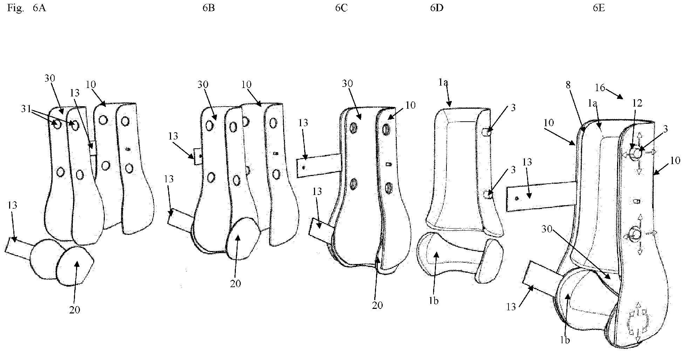

Die Fig. 6a bis 6e zeigen eine Ausführungsform, bei der die Einlage 1 in Einlagesegmente 1a, 1b unterteilt ist, von denen ein Einlagesegment 1b passend zum Schalensegment 20 ausgebildet ist und an diesem mit zwischenliegender Zwischenlage 30 angeordnet ist, wobei bevorzugt das Einlagesegment 1b auf der einen Seite der Zwischenlage 30 angebracht und passend das auf der gegenüberliegenden Seite der Zwischenlage 30 angebrachte Schalensegment 20 überdeckt. In dieser Ausführungsform ist bevorzugt, dass das Einlagesegment 1b, das vom Schalensegment 20 umfasst ist, in einem Hüllensegment als formbare Masse ein dilatantes Fluid, bevorzugt borhaltiges Polydimethylsiloxan enthält.

Auch in dieser Ausführungsform ist das Schalensegment 20 mit dem daran befestigten Einlagesegment 1b durch die Biegsamkeit der Zwischenlage 30 gegenüber der Schale 10 beweglich.

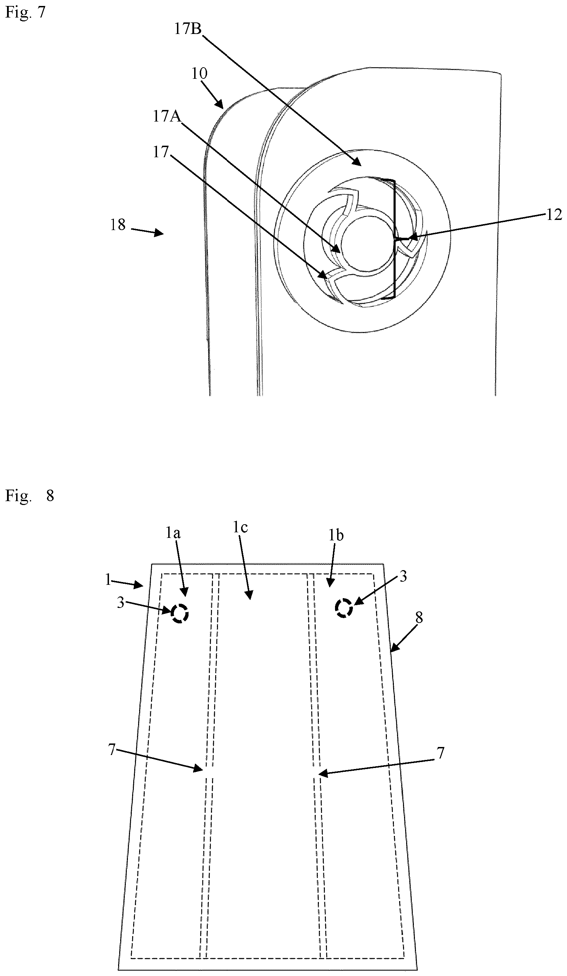

Fig. 7 zeigt einen ersten Endabschnitt 18 einer Schale 1 mit einer Halteausnehmung 12, in der sich Federelemente 17 über den Querschnitt der Halteausnehmung 12 bis zu einer Halteaufnahme 17A erstrecken. Die Halteaufnahme 17A, hier ringförmig ausgebildet, weist einen Innenquerschnitt auf, in den ein Haltevorsprung 3 einer Einlage 1 geklemmt werden kann. Die Federelemente 17, hier zur Radialen der Halteausnehmung 12 bogenförmig und geknickt dargestellt, können z.B. aus Kunststoff oder Federstahl bestehen. Optional sind die Federelemente 17 einstückig mit einem Federrahmen 17B und der Halteaufnahme 17A ausgebildet, z.B. aus Kunststoff, wobei der Federrahmen 17B an der Schale 10 befestigt ist.

Fig. 8 zeigt eine in Einlagensegmente 1a, 1b, 1c unterteilte Einlage 1, die durch Segmente 1a, 1b, 1c einer Hülle gebildet werden, die durch eine unterbrochene Schweißnaht 7 voneinander beabstandet sind, wobei die unterbrochene Schweißnaht 7 eine Leitung bildet, die die Einlagensegmente 1a, 1b, 1c miteinander verbindet. Daher können die Einlagensegmente 1a, 1b, 1c durch ein einziges gemeinsames Ventil 4 vakuumiert bzw. mit Umgebungsdruck beaufschlagt werden. Die Umfangskante 8 der Einlage 1 ist bevorzugt abschnittsweise oder vollständig durch eine Schweißnaht gebildet.

### Bezugszeichen:

1 Einlage
1a, 1b, 1c Einlagesegment
2 Außenwand
3 Haltevorsprung
4 Ventil
5 erster Endabschnitt der Einlage
6 zweiter Endabschnitt der Einlage
7 unterbrochene Schweißnaht
8 Umfangskante
10 Schale
11 Schenkel
12 Halteausnehmung
13 Lasche
14 Vorsprung
15 Ausnehmung
16 Längsachse
17 Federelement
17A Halteaufnahme
17B Federrahmen
18 erster Endabschnitt der Schale
19 zweiter Endabschnitt der Schale
20 Schalensegment
21 Haltevorsprung des Schalensegments
22 Lasche des Schalensegments
23 Schenkel des Schalensegments
30 Zwischenlage
31 Ausnehmungen der Zwischenlage

## Patentansprüche

1. Gamasche mit einer Einlage (1), die von einer Schale (10) umfasst ist, bei der die Einlage (1) eine elastische Hülle ist, die eine formbare Masse enthält,
wobei die Einlage (1) an zumindest einem Bereich ihrer Außenwand zumindest einen Haltevorsprung (3) aufweist,
die Schale zwei gegenüberliegende Schenkel (11) aufweist, die zumindest eine passend zu dem Haltevorsprung (3) der Einlage (1) angeordnete Halteausnehmung (12) aufweist, die einen größeren Querschnitt als der Haltevorsprung (3) aufweist, mit zumindest einer Lasche (13), die lösbar an gegenüberliegenden Schenkeln (11) der Schale (10) befestigbar ist, um den zwischen den Schenkeln (11) liegenden Freiraum zu überbrücken und die gegenüberliegenden Schenkel (11) aufeinander zu zu spannen, **gekennzeichnet dadurch, dass** zumindest einer der Haltevorsprünge (3) federnd schwimmend mittels zumindest eines Federelements (17), das sich in der Halteausnehmung (12) bis zu einem Haltevorsprung (3) erstreckt, in je einer Halteausnehmung (12) gehalten ist.

2. Gamasche nach Anspruch 1, **dadurch gekennzeichnet, dass** die Einlage (1) genau zwei Haltevorsprünge (3) aufweist, die an einem ersten Endabschnitt der Einlage (1) angeordnet sind, der sich über maximal 30% der Länge der Einlage (1) entlang der Längserstreckung der Schale (10) erstreckt, und die Halteausnehmungen (12) in einem ersten Endabschnitt (18) der Schale (10) angeordnet sind und dadurch der zweite Endabschnitt (19) der Schale (10) eingerichtet ist, in einen Abstand von dem dem ersten Endabschnitt der Einlage gegenüberliegenden zweiten Endabschnitt der Einlage (1) beweglich zu sein, wobei optional die Schale (10) in ihrem dem ersten Endabschnitt (18) gegenüberliegenden zweiten Endabschnitt (19) einen größeren Innenquerschnitt als im ersten Endabschnitt (18) aufweist.

3. Gamasche nach einem der voranstehenden Ansprüche, **dadurch gekennzeichnet, dass** die Einlage in zumindest ein Einlagensegment (1c) mit weniger formbarer Masse oder ohne formbare Masse und angrenzend zumindest ein Einlagensegment (1a, 1b) unterteilt ist, das mehr formbare Masse enthält, wobei das Einlagensegment (1c) mit weniger oder ohne formbare Masse und das zumindest eine angrenzende Einlagensegment (1a, 1b) durch eine unterbrochene Schweißnaht (7) verbunden sind.

4. Gamasche nach einem der voranstehenden Ansprüche, **dadurch gekennzeichnet, dass** die Hülle evakuierbar ist, ein Ventil (4) aufweist und die formbare Masse durch lose Partikel gebildet wird.

5. Gamasche nach einem der voranstehenden Ansprüche, **dadurch gekennzeichnet, dass** die formbare Masse durch ein dilatantes Fluid, optional in Mischung mit Partikeln, gebildet wird.

6. Gamasche nach einem der voranstehenden Ansprüche, **dadurch gekennzeichnet, dass** in der Schale (10) ein Schalensegment (20) beweglich angeordnet ist, das einen endständigen Abschnitt (1b) der Einlage (1) an deren zweiten Endabschnitt (6) umfasst, der ihrem ersten Endabschnitt (5) gegenüber liegt, in dem die Haltevorsprünge (3) angeordnet sind.

7. Gamasche nach einem der voranstehenden Ansprüche, **dadurch gekennzeichnet, dass** zwischen der Schale (10) und der Einlage (1) eine Zwischenlage (30) angeordnet ist, die zumindest eine Ausnehmung (31) aufweist, die um einen Haltevorsprung (3) angeordnet ist.

8. Gamasche nach einem der voranstehenden Ansprüche, **dadurch gekennzeichnet, dass** sich die Halteausnehmungen (12) von der konkaven Innenseite der Schale (10) erstrecken, wobei die gegenüberliegende konvexe Außenseite der Schale (10) eine geschlossene Oberfläche hat.

9. Gamasche nach einem der voranstehenden Ansprüche, **dadurch gekennzeichnet, dass** die Einlage (1) eine elastische Hülle ist, innerhalb derer eine mit Partikeln gefüllte gasdurchlässige Innenlage angeordnet ist, die in mit Partikeln gefüllte Segmente (1a, 1b, 1c) unterteilt ist.

10. Gamasche nach einem der Ansprüche 1 bis 9, **dadurch gekennzeichnet, dass** die Einlage (1) in miteinander verbundene Einlagensegmente (1a, 1b) unterteilt ist, die jeweils Segmente einer Hülle sind, wobei die Einlagensegmente (1a, 1b) durch eine Leitung miteinander verbunden sind und ein gemeinsames Ventil (4) aufweisen, oder voneinander getrennt sind und jeweils ein separates Ventil (4) aufweisen.

11. Gamasche nach einem der voranstehenden Ansprüche, **dadurch gekennzeichnet, dass** die Einlage (1) in Einlagensegmente (1a, 1b) unterteilt ist, von denen zumindest eines ein dilatantes Fluid als formbare Masse enthält.

12. Gamasche nach einem der Ansprüche 6 bis 11, **dadurch gekennzeichnet, dass** ein Einlagesegment (1b) von dem Schalensegment (20) umfasst ist.

13. Gamasche nach einem der Ansprüche 6 bis 12, **dadurch gekennzeichnet, dass** das Schalensegment (20) zumindest einen Haltevorsprung (21) aufweist, der in eine Ausnehmung (15) der Schale (10) ragt und das Schalensegment (20) beweglich in der Schale (10) führt.

14. Gamasche nach einem der voranstehenden Ansprüche, **dadurch gekennzeichnet, dass** jeweils eine Schale (10) mit zumindest zwei Einlagen (1) ein System bildet, bei dem die Einlagen (1) gegeneinander austauschbar sind und jede Einlage (1) als formbare Masse eine unterschiedliche Füllung von Partikeln und/oder dilatantem Fluid aufweist.

15. Gamasche nach einem der voranstehenden Ansprüche, **dadurch gekennzeichnet, dass** die Einlage (1) an ihrer umlaufenden Umfangskante (8) von der sie umfassenden Schale (10) um 1 bis 15 mm überragt wird.

## Claims

1. Gaiter with an insert (1) which is encompassed by a shell (10), in which the insert (1) is an elastic sleeve containing a moldable mass,
wherein the insert (1) has at least one retaining projection (3) on at least one region of its outer wall,
the shell having two opposite legs (11) which comprise at least one retaining recess (12) arranged to match the retaining projection (3) of the insert (1) and having a cross-section larger than the retaining projection (3),
with at least one tab (13) which can be releasably attached to opposite legs (11) of the shell (10) in order to bridge the free space lying between the legs (11) and to tension the opposite legs (11) towards one another, **characterized in that** at least one of the retaining projections (3) is held in a resiliently floating manner in one retaining recess each (12) by means of at least one spring element (17) which extends in the retaining recess (12) up to a retaining projection (3).

2. Gaiter according to claim 1, **characterized in that** the insert (1) has exactly two retaining projections (3) which are arranged at a first terminal section of the insert (1), which first terminal section extends over a maximum of 30% of the length of the insert (1) along the longitudinal extension of the shell (10), and the retaining recesses (12) are arranged in a first terminal section (18) of the shell (10) and thereby the second terminal section (19) of the shell (10) is set up to be movable into a distance from the second terminal section of the insert (1) lying opposite to the first terminal section of the insert, wherein optionally the shell (10) in its second terminal section (19) lying opposite to the first terminal section (18) has a larger internal cross-section than in the first terminal section (18).

3. Gaiter according to one of the preceding claims, **characterized in that** the insert is divided into at least one insert segment (1c) with less moldable mass or without moldable mass and adjacent to at least one insert segment (1a, 1b) containing more moldable mass, the insert segment (1c) with less or without moldable mass and the at least one adjacent insert segment (1a, 1b) being connected by an interrupted weld seam (7).

4. Gaiter according to one of the preceding claims, **characterized in that** the sleeve is evacuable, has a valve (4) and the moldable mass is formed by loose particles.

5. Gaiter according to one of the preceding claims, **characterized in that** the moldable mass is formed by a dilatant fluid, optionally in admixture with particles.

6. Gaiter according to one of the preceding claims, **characterized in that** a shell segment (20) is movably arranged in the shell (10), which shell segment (20) encompasses a terminal section (1b) of the insert (1) at its second terminal section (6) lying opposite to its first terminal section (5) in which the retaining projections (3) are arranged.

7. Gaiter according to one of the preceding claims, **characterized in that** an intermediate layer (30) is arranged between the shell (10) and the insert (1), which intermediate layer (30) has at least one recess (31) arranged around a retaining projection (3).

8. Gaiter according to of the preceding claims, **characterized in that** the retaining recesses (12) extend from the concave inner side of the shell (10), wherein the opposite convex outer side of the shell (10) has a closed surface.

9. Gaiter according to one of the preceding claims, **characterized in that** the insert (1) is an elastic sleeve within which a gas-permeable inner layer filled with particles is arranged, which inner layer is subdivided into segments (1a, 1b, 1c) filled with particles.

10. Gaiter according to one of claims 1 to 9, **characterized in that** the insert (1) is divided into interconnected insert segments (1a, 1b), which are each segments of a sleeve, wherein the insert segments (1a, 1b) are interconnected by a conduit and have a common valve (4), or are separated from each other and each have a separate valve (4).

11. Gaiter according to one of the preceding claims, **characterized in that** the insert (1) is divided into insert segments (1a, 1b), at least one of which contains a dilatant fluid as a moldable mass.

12. Gaiter according to one of claims 6 to 11, **characterized in that** an insert segment (1b) is encompassed by the shell segment (20).

13. Gaiter according to one of claims 6 to 12, **characterized in that** the shell segment (20) has at least one retaining projection (21) which projects into a recess (15) of the shell (10) and movably guides the shell segment (20) in the shell (10).

14. Gaiter according to one of the preceding claims, **characterized in that** in each case one shell (10) with at least two inserts (1) forms a system in which the inserts (1) are interchangeable with one another and each insert (1) has a different filling of particles and/or dilatant fluid as a moldable mass.

15. Gaiter according to one of the preceding claims, **characterized in that** the insert (1) is surpassed by 1 to 15 mm at its circumferential edge (8) by the shell (10) encompassing it.

## Revendications

1. Guêtre avec un insert (1) qui est entouré d'une coque (10), dans laquelle l'insert (1) est une enveloppe élastique qui contient une masse déformable
l'insert (1) présentant, sur au moins une zone de sa paroi extérieure, au moins une saillie de retenue (3),
la coque présente deux branches (11) opposées, qui présentent au moins un évidement de retenue (12) disposé de manière adaptée à la saillie de retenue (3) de l'insert (1), qui présente une section transversale plus grande que la saillie de retenue (3),
avec au moins une patte (13) qui peut être fixée de manière amovible sur des branches (11) opposées de la coque (10), afin de combler l'espace libre situé entre les branches (11) et de serrer les branches (11) opposées l'une contre l'autre, **caractérisé en ce qu'**au moins l'une des saillies de retenue (3) est maintenue de manière flottante et élastique dans un évidement de retenue (12) respectif au moyen d'au moins un élément élastique (17) qui s'étend dans l'évidement de retenue (12) jusqu'à une saillie de retenue (3).

2. Guêtre selon la revendication 1, **caractérisée en ce que** l'insert (1) présente exactement deux saillies de retenue (3) qui sont disposées sur une première section d'extrémité de l'insert (1) qui s'étendent sur au maximum 30% de la longueur de l'insert (1) le long de l'extension longitudinale de la coque (10), et les évidements de retenue (12) sont disposés dans une première section d'extrémité (18) de la coque (10) et la deuxième section d'extrémité (19) de la coque (10) est ainsi aménagée, être mobile à une distance de la deuxième partie d'extrémité de l'insert (1) opposée à la première partie d'extrémité de l'insert, dans lequel, en option, la coque (10) présente dans sa deuxième partie d'extrémité (19) opposée à la première partie d'extrémité (18) une section transversale intérieure plus grande que dans la première partie d'extrémité (18).

3. Guêtre selon l'une des revendications précédentes, **caractérisée en ce que** l'insert est divisé en au moins un segment d'insert (1c) avec moins de masse malléable ou sans masse malléable et adjacent au moins un segment d'insert (1a, 1b) contenant plus de masse malléable, le segment d'insert (1c) avec moins de masse malléable ou sans masse malléable et le au moins un segment d'insert adjacent (1a, 1b) étant reliés par un cordon de soudure discontinu (7).

4. Guêtre selon l'une des revendications précédentes, **caractérisée en ce que** l'enveloppe peut être mise sous vide, comporte une valve (4) et la masse malléable est formée par des particules libres.

5. Guêtre selon l'une quelconque des revendications précédentes, **caractérisée en ce que** la masse malléable est formée par un fluide dilatant, éventuellement en mélange avec des particules.

6. Guêtre selon l'une quelconque des revendications précédentes, **caractérisée en ce qu'**un segment de coque (20) est disposé de manière mobile dans la coque (10) et comprend une partie d'extrémité (1b) de l'insert (1) au niveau de sa deuxième partie d'extrémité (6), opposée à sa première partie d'extrémité (5), dans laquelle sont disposées les saillies de retenue (3).

7. Guêtre selon l'une des revendications précédentes, **caractérisée en ce qu'**entre la coque (10) et l'insert (1) est disposée une couche intermédiaire (30) qui présente au moins un évidement (31) qui est disposé autour d'une saillie de retenue (3).

8. Guêtre selon l'une des revendications précédentes, **caractérisée en ce que** les évidements de retenue (12) s'étendent depuis la face interne concave de la coque (10), la face externe convexe opposée de la coque (10) ayant une surface fermée.

9. Guêtre selon l'une des revendications précédentes, **caractérisée en ce que** l'insert (1) est une enveloppe élastique à l'intérieur de laquelle est disposée une couche intérieure perméable aux gaz, remplie de particules, qui est divisée en segments (1a, 1b, 1c) remplis de particules.

10. Guêtre selon l'une des revendications 1 à 9, **caractérisée en ce que** l'insert (1) est divisé en segments d'insert (1a, 1b) reliés entre eux, qui sont chacun des segments d'une enveloppe, les segments d'insert (1a, 1b) étant reliés entre eux par un conduit et présentant une valve commune (4), ou étant séparés les uns des autres et présentant chacun une valve séparée (4).

11. Guêtre selon l'une des revendications précédentes, **caractérisée en ce que** l'insert (1) est divisé en segments d'insert (1a, 1b), dont au moins un contient un fluide dilatant en tant que masse malléable.

12. Guêtre selon l'une des revendications 6 à 11, **caractérisée en ce qu'**un segment d'insertion (1b) est compris dans le segment de coque (20).

13. Guêtre selon l'une des revendications 6 à 12, **caractérisée en ce que** le segment de coque (20) présente au moins une saillie de retenue (21) qui fait saillie dans un évidement (15) de la coque (10) et guide le segment de coque (20) de manière mobile dans la coque (10).

14. Guêtre selon l'une des revendications précédentes, **caractérisée en ce que** respectivement une coque (10) avec au moins deux inserts (1) forme un système dans lequel les inserts (1) sont interchangeables et chaque insert (1) présente, en tant que masse malléable, un remplissage différent de particules et/ou de fluide dilatant.

15. Guêtre selon l'une des revendications précédentes, **caractérisée en ce que** l'insert (1) est dépassé de 1 à 15 mm sur son bord périphérique par la coque (10) qui l'entoure.
